# FASCICULE DE BREVET EUROPEEN

(11) **EP 0 630 428 B1**
(45) Date de publication et mention de la délivrance du brevet: **08.11.1995**
(21) Numéro de dépôt: 93918756.3
(22) Date de dépôt: 11.03.1993
(51) Int. Cl.: C25B 3/04, C07D 305/14

(54) **PROCEDE DE PREPARATION DE DERIVES DU TAXANE**
VERFAHREN ZUR HERSTELLUNG VON TAXANDERIVATEN
PROCESS FOR THE PREPARATION OF TAXANE DERIVATIVES

(30) Priorité: 13.03.1992 FR 9203000
(43) Date de publication de la demande: 28.12.1994
(73) Titulaire: RHONE-POULENC RORER S.A., 92160 Antony (FR)
(72) Inventeur: DEPREZ, Dominique, F-91310 Leuville-sur-Orge (FR); PULICANI, Jean-Pierre, F-92160 Antony (FR)
(74) Mandataire: Pilard, Jacques
(86) Numéro de dépôt international: FR9300243
(87) Numéro de publication internationale: WO9318210

(56) Documents cités:
- EP-A- 0 253 738
- EP-A- 0 336 840
- WO-A-92/01691
- PATENT ABSTRACTS OF JAPAN, vol. 10, no. 36 (C-328)(2093), 13 Février 1986; & JP-A-60 187 689

## Description

La présente invention concerne un procédé de préparation de dérivés du taxane de formule générale : 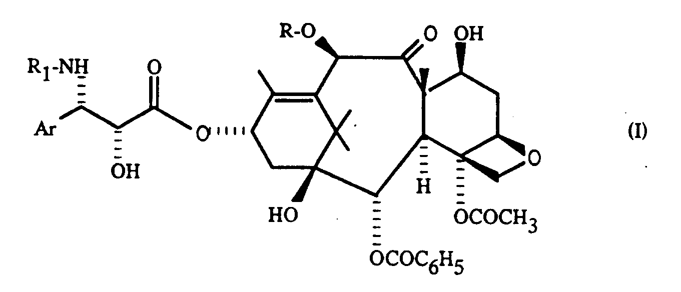
qui présentent des propriétés antitumorales et antileucémiques remarquables.

Dans la formule générale (I),
R réprésente un atome d'hydrogène ou un radical acétyle, R₁ réprésente un radical benzoyle ou un radical R₂-O-CO- dans lequel R₂ représente un radical alcoyle, alcényle, alcynyle, cycloalcoyle, cycloalcényle, bicycloalcoyle, phényle ou hétérocyclyle azoté, et Ar représent un radical aryle.

Plus particulièrement, R représente un atome d'hydrogène ou un radical acétyle et R₁ représente un radical benzoyle ou un radical R₂-O-CO- dans lequel R₂ représente :
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 10 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les radicaux alcoyles contenant 1 à 4 atomes de carbone ou alcoyloxy contenant 1 à 4 atomes de carbone,
- ou un radical hétérocyclyle azoté saturé ou non saturé contenant 5 ou 6 chaînons éventuellement substitué par un ou plusieurs radical alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone, et
Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, aryles, arylalcoyles, alcoxy, alcoylthio, aryloxy, arylthio, hydroxy, hydroxyalcoyle, mercapto, formyle, acylamino, aroylamino, alcoxycarbonylamino, amino, alkylamino, dialkylamino, carboxy, alcoxycarbonyle, carbamoyle, dialcoylcarbamoyle, cyano et trifluorométhyle, étant entendu que les radicaux alcoyles et les portions alcoyles des autres radicaux contiennent 1 à 4 atomes de carbone et les radicaux aryles sont les radicaux phényles ou α- ou β-naphtyles.

D'un intérêt tout particulier sont les produits de formule générale (I) dans laquelle R représente un atome d'hydrogène ou un radical acétyle, R₁ représente un radical benzoyle ou t.butoxycarbonylamino et Ar représente un radical phényle.

Les produits de formule générale (I) dans laquelle R₁ représente un radical benzoyle correspondent au taxol et au désacétyl-10 taxol et les produits de formule générale (I) dans laquelle R₁ représente un radical t.butoxycarbonyle correspondent à ceux qui font l'objet du brevet européen EP 0 253 738.

Selon les procédés qui sont décrits par exemple dans les brevets européens EP 0 253 738, EP 0 336 840 et EP 0 336 841 les produits de formule générale (I) sont obtenus après remplacement par des atomes d'hydrogène des groupements protecteurs R', G₁ et G₂ du produit de formule générale : 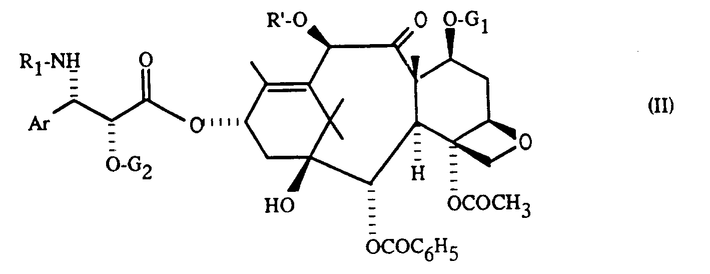
dans laquelle Ar et R₁ sont définis comme précédemment, R' représente un radical acétyle ou un groupement protecteur de la fonction hydroxy choisi parmi les radicaux trichloro-2,2,2 éthoxycarbonyle ou trialkylsilyle dont chaque partie alkyle contient 1 à 3 atomes de carbone, G₁ représente un groupement protecteur de la fonction hydroxy choisi parmi les radicaux trichloro-2,2,2 éthoxycarbonyle ou trialkylsilyle dont chaque partie alkyle contient 1 à 3 atomes de carbone, et G₂ représente un atome d'hydrogène ou un groupement protecteur de la fonction hydroxy choisi parmi les radicaux méthoxyméthyle, éthoxy-1 éthyle, benzyloxyméthyle, (β-triméthylsilyléthoxy) méthyle, tétrahydropyrannyle, trichloro-2,2,2 éthoxycarbonyle ou trichloro-2,2,2 éthoxyméthyle.

Selon la nature des groupements protecteurs, leur remplacement par des atomes d'hydrogène peut être effectué au moyen de zinc en présence d'un acide minéral ou organique tel que l'acide chlorhydrique ou l'acide acétique éventuellement en solution dans un alcool aliphatique contenant 1 à 3 atomes de carbone, en particulier lorsque au moins un des groupements protecteurs représente un radical trichloro-2,2,2 éthoxycarbonyle, ou au moyen d'un acide tel que l'acide chlorhydrique dans un alcool aliphatique contenant 1 à 3 atomes de carbone à une température voisine de 0°C lorsque au moins un des groupements protecteurs représente un radical trialkylsilyle.

Il a maintenant été trouvé, et c'est ce qui fait l'objet de la présente invention, que les produits de formule générale (I) peuvent être obtenus par réduction électrolytique d'un produit de formule générale (II) dans laquelle Ar et R₁ sont définis comme précédemment, R' représente un radical acétyle ou un radical trichloro-2,2,2 éthoxycarbonyle, G₁ représente un radical trichloro-2,2,2 éthoxycarbonyle et G₂ représente un atome d'hydrogène, c'est-à-dire le produit de formule générale (IIa).

Selon l'invention, le remplacement des groupements protecteurs des fonctions hydroxy par un atome d'hydrogène est effectué par réduction électrolytique selon la réaction suivante : 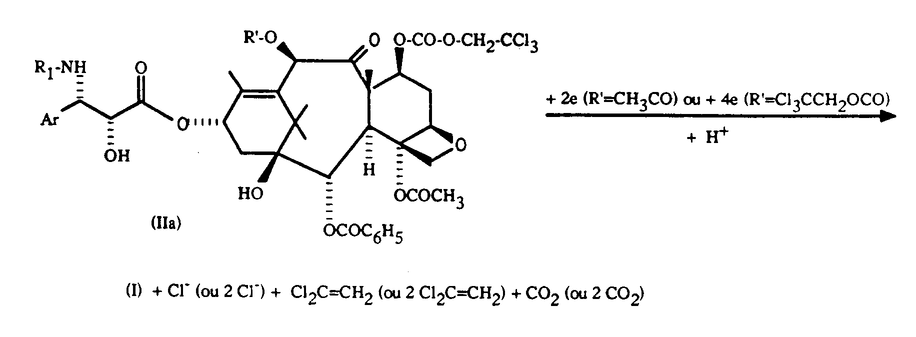
La réduction électrolytique à partir du produit de formule générale (IIa) est réalisée dans un électrolyseur contenant un catholyte constitué d'un électrolyte support dans lequel est dissous le produit de formule générale (IIa) à une concentration comprise entre 0,1 et 40 g/litre.

La quantité d'électricité nécessaire pour effectuer la réduction d'un produit de formule générale (IIa) en produit de formule générale (I) est de 386.000 coulombs par molécule lorsque R' représente un radical trichloro-2,2,2 éthoxycarbonyle et elle est de 193.000 coulombs par molécule lorsque R' représente un radical acétyle.

Préférentiellement, la réduction s'effectue dans un électrolyseur à diaphragme.

Selon un mode de mise en oeuvre du procédé selon l'invention, la réduction électrolytique est effectuée dans un électrolyseur comportant une cathode, un compartiment cathodique, un diaphragme séparateur, un compartiment anodique et une anode dont les caractéristiques sont les suivantes :
a) la cathode est constituée d'un matériau conducteur de l'électricité sur lequel la réduction a lieu à un potentiel supérieur à celui de la réduction du solvant ou de l'un des constituants de l'électrolyte support, ou à un potentiel tel que la réduction du solvant ou de l'un des constituants de l'électrolyte support n'est pas suffisamment importante pour gêner la réduction du produit,
b) le compartiment cathodique contient le catholyte qui est constitué d'une solution du produit de formule générale (IIa) dans un milieu organique ou hydroorganique dont le pH apparent est compris entre 4 et 7 et d'un électrolyte support,
c) le diaphragme séparateur est constitué d'un matériau poreux tel qu'une plaque, un manchon ou une bougie de verte fritté ou de porcelaine ou par une membrane échangeuse d'ions, de préférence une membrane échangeuse de cations,
d) le compartiment anodique contient l'anolyte constitué de préférence par le même solvant ou mélange de solvants et le même électrolyte support que celui qui est utilisé dans le compartiment cathodique,
e) l'anode est constituée d'un matériau conducteur de l'électricité dont la nature n'est pas essentielle à la mise en oeuvre du procédé.

Généralement, l'anode est constituée par un matériau conducteur de l'électricité inattaquable dans les conditions de l'électrolyse tel que par exemple le platine poli, massif ou sur support conducteur, le graphite ou le carbone vitreux et la cathode est constituée d'un matériau conducteur de l'électricité tel que le mercure, le zinc, le plomb ou le carbone vitreux.

De préférence la cathode est constituée par une nappe de mercure.

L'électrolyte support est constituée d'un sel alcalin ou d'un sel d'ammonium quaternaire, tel que l'acétate de sodium en présence d'acide acétique ou l'acétate de tétraéthylammonium, soluble dans le solvant ou le mélange hydroorganique. Généralement, on utilise des solvants qui solubilisent facilement les produits de formule générale (I) et (IIa) et qui permettent un isolement facile du produit de formule générale (I) tels que les alcools (méthanol), les nitriles (acétonitrile).

Le pH doit être compatible avec la stabilité du substrat et il est de préférence compris entre 4 et 6 et il peut être maintenu constant au cours de l'électrolyse par addition d'un acide.

La nature du diaphragme séparant l'anolyte du catholyte n'est pas une caractéristique essentielle de l'invention. C'est ainsi qu'on peut utiliser tout diaphragme de type connu, constitué par un matériau poreux comme le verte fritté, la porcelaine avec ou sans gel conducteur limitant la diffusion des réactifs ou par des membranes échangeuses d'ions de préférence échangeuses de cations. Les membranes peuvent être de type homogène ou hétérogène et elles peuvent éventuellement être renforcées par une trame. De préférence sont utilisées des membranes qui ne gonflent pas, qui ne gaufrent pas et qui sont stables en présence des divers constituants de l'anolyte et du catholyte.

Selon un mode de mise en oeuvre préféré de l'invention l'anode, la cathode et le diaphragme séparateur sont disposés selon des plans parallèles de préférence verticaux ou horizontaux dans le cas d'une cathode constituée par une nappe de mercure. En outre plusieurs électrolyseurs élémentaires peuvent être associés à la manière des filtre-presses.

La température du bain d'électrolyse est généralement comprise entre 0 et 30°C.

L'électrolyse peut être effectuée à densité de courant constante ou à un potentiel d'électrode imposé. De préférence, l'électrolyse est réalisée à densité de courant constante comprise entre 0,5 et 10 A/dm2.

Lorsque l'électrolyse est effectuée à potentiel contrôlé, celui-ci est fixé à environ -1,4 volt par rapport à une électrode de référence au calomel.

La quantité théorique d'électricité mise en oeuvre est de 193.000 coulombs par mole à partir d'un produit de formule générale (IIa) pour lequel R' représente un radical acétyle et est de 386.000 coulombs par mole à partir d'un produit de formule générale (IIa) pour lequel R' représente une radical trichloro-2,2,2 éthoxycarbonyle. Pratiquement la quantité d'électricité utilisée peut varier entre 1,5 fois et 8 fois la quantité théorique.

Habituellement le catholyte circule en circuit fermé, par exemple sous l'action d'une pompe. Le circuit peut en outre comprendre des dispositifs annexes tels qu'échangeurs de température ou vases d'expansion ; un tel vase d'expansion permet en particulier d'alimenter le catholyte en produit de formule générale (IIa) et permet également d'effectuer un soutirage pour l'extraction du produit de formule générale (I).

L'anolyte peut également être soumis à une circulation. Selon un mode préférentiel de réalisation de l'invention, le circuit du catholyte est similaire à celui de l'anolyte, ce qui permet d'équilibrer les pressions de part et d'autre du diaphragme séparateur.

Selon un autre mode de mise en oeuvre particulier de l'invention, on dispose des intercalaires dans les compartiments anodique et cathodique. Ces intercalaires servent à éviter, d'une part, les déformations de la membrane échangeuse d'ions et, d'autre part, les contacts de cette membrane avec les électrodes. Ils servent également à améliorer l'homogénéité de concentration du catholyte.

En l'absence d'intercalaire, la vitesse de circulation du catholyte dans le compartiment cathodique est habituellement supérieure à 10 cm/s, de préférence supérieure à 50 cm/s. Lorsque l'on utilise un intercalaire, la vitesse apparente du catholyte (vitesse dans le compartiment cathodique supposé sans intercalaire) est habituellement supérieure à 1 cm/s, de préférence supérieure à 10 cm/s.

Selon un autre mode de mise en oeuvre de l'invention, la cellule peut être constituée simplement d'un récipient, parallélépipédique ou cylindrique, en une matière inerte vis-à-vis des constituants des électrolytes. Ce récipient contient l'électrode de travail dont la nature est la même que celle définie pour le premier genre de cellule. La forme de cette électrode de travail est adaptée à la forme du récipient.

D'une façon générale, toute cellule électrolytique comportant une anode et une cathode séparées par un ou plusieurs diaphragmes assurant la conductibilité ionique est susceptible d'être employée, la disposition des éléments n'étant pas essentielle à la mise en oeuvre du procédé.

Le produit de formule générale (I) obtenu par la mise en oeuvre du procédé selon l'invention est séparé par application des méthodes habituelles.

Les produits de formule générale (IIa) peuvent être obtenus dans les conditions décrites dans le brevet européen EP 0 253 738.

Les produits de formule générale (IIa) peuvent aussi être obtenus par condensation d'un dérivé d'une β-arylisosérine de formule générale : 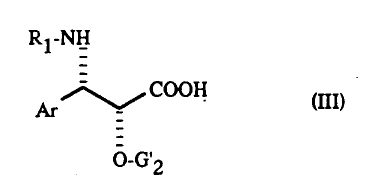
dans laquelle Ar et R₁ sont définis comme précédemment et G'₂ représente un groupement protecteur de la fonction hydroxy sur un dérivé de la baccatine III ou de la désacétyl-10 baccatine III de formule générale : 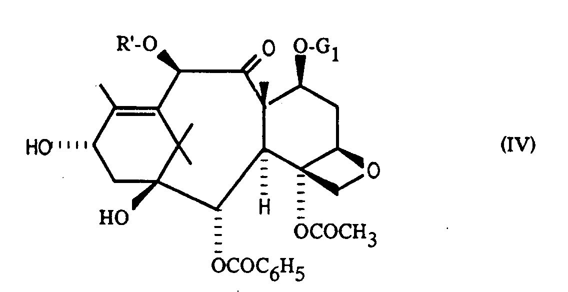
dans laquelle R' et G₁ sont définis comme précédemment, en opérant dans les conditions décrites dans les brevets européens EP 0 336 840 et EP 0 336 841, suivie du remplacement du groupement protecteur G'₂ par un atome d'hydrogène en opérant en milieu acide.

Les produits de formule générale (IIa) peuvent également être obtenus par condensation d'un acide de formule générale : 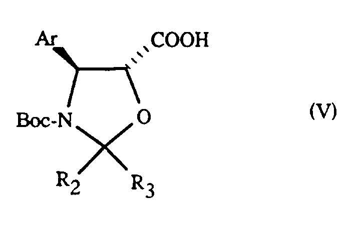
dans laquelle Ar est défini comme précédemment, Boc représente le radical t.butoxycarbonyle et R₂ et R₃, identiques ou différents, représentent un atome d'hydrogène ou un radical alcoyle contenant 1 à 4 atomes de carbone, éventuellement substitué par un ou plusieurs radicaux aryles (phényles), ou aryle (phényle) ou bien R₂ et R₃ forment ensemble avec l'atome de carbone auquel ils sont liés un cycle ayant de 4 à 7 chaînons, sur un dérivé de la baccatine III ou de la désacétyl-10 baccatine III de formule générale (IV) pour obtenir le produit de formule générale : 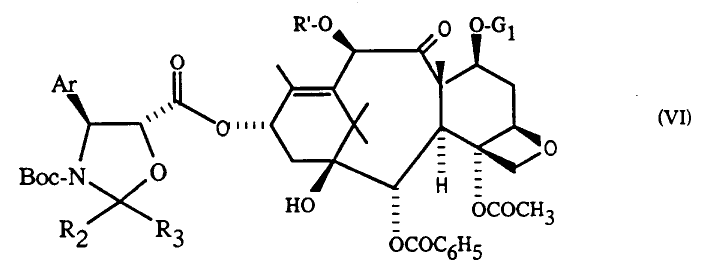
dans laquelle Ar, Boc, R', G₁, R₂ et R₃ sont définis comme précédemment, sur lequel on fait réagir un acide minéral ou organique éventuellement dans un alcool dans des conditions qui sont sans effet sur les groupements protecteurs R' et G₁ pour obtenir un produit de formule générale : 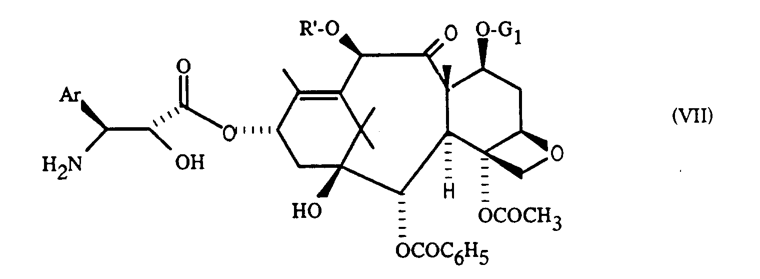
dans laquelle Ar, R' et G₁ sont définis comme précédemment, sur lequel on fait réagir un halogénure de benzoyle ou un dérivé réactif de formule générale :

R₂-O-CO-X (VIII)

dans laquelle R₂ est défini comme précédemment et X représente un atome d'halogène (fluor, chlore) ou un reste -O-R₂ ou -O-CO-O-R₂ pour obtenir le produit de formule générale (IIa).

Les exemples suivants montrent comment l'invention peut être mise en pratique.

### EXEMPLE 1

On procède à la réduction électrolytique du tert.butoxycarbonylamino-3 phényl-3 hydroxy-2 propionate-(2R,3S) d'acétoxy-4 benzoyloxy-2α époxy-5β,20 hydroxy-1 oxo-9 bis-(trichloro-2,2,2 éthoxycarbonyloxy)-7β,10β taxène-11 yle-13α dans une cellule d'électrolyse possédant les caractéristiques suivantes :
- la cellule est un vase en verre de 25 cm3 divisé en deux compartiments par une membrane échangeuse de cations,
- la cathode est une nappe de mercure dont la surface utile est de 4 cm2,
- l'anode est une grille de platine,
- l'électrode de référence est une électrode au calomel.

Dans le compartiment cathodique, on introduit 10 cm3 d'une solution contenant :

| | |
|---|---|
| - tert.butoxycarbonylamino-3 phényl-3 hydroxy-2 propionate-(2R,3S) d'acétoxy-4 benzoyloxy-2α époxy-5β,20 hydroxy-1 oxo-9 bis-(trichloro-2,2,2 éthoxycarbonyloxy)-7β,10β taxène-11 yle-13α | 53,3 mg |
| - acide acétique | 1M |
| - acétate de sodium (CH₃COONa,3H₂O) | 0,1M |
| - méthanol | q.s.p. 10 cm3 |

Dans la compartiment anodique, on introduit 10 cm3 du même mélange ne contenant pas le substrat.

Après désaération pendant 10 minutes par barbotage d'un courant d'argon, le potentiel de la cathode est fixé à -1,4 volt par rapport à l'électrode de référence.

On électrolyse la solution pendant le temps nécessaire au passage de 30 coulombs.

A l'électrolysat séparé, on ajoute 9 cm3 d'eau permutée. Après avoir chassé le solvant sous pression réduite à une température inférieure à 35°C, on extrait la phase aqueuse restante par 3 fois 20 cm3 de dichlorométhane puis on rince la phase organique par 2 fois 20 cm3 de tampon phosphate de sodium (pH 7,4). Après évaporation du solvant sous pression réduite à une température inférieure à 35°C, on obtient, après séparation par chromatographie en couche mince préparative (gel de silice : 0,25 mm d'épaisseur ; milieu : dichlorométhane-méthanol (94-6 en volumes) avec un rendement de 50 %, le tert.butoxycarbonylamino-3 phényl-3 hydroxy-2 propionate-(2R,3S) d'acétoxy-4 benzoyloxy-2α époxy-5β,20 trihydroxy-1,7β,10β oxo-9 taxène-11 yle-13α (ou "Taxotère") dont les caractéristiques sont identiques à celles du produit qui est décrit dans le brevet européen EP 0 253 738.

### EXEMPLE 2

On effectue l'électrolyse du substrat utilisé dans l'exemple 1 dans une cellule d'électrolyse de 25 cm3 ne contenant pas de membrane séparatrice et comportant :
- une cathode constituée d'une nappe de mercure dont la surface utile est de 4 cm2,
- une électrode de référence au calomel,
- une anode constituée d'une grille de platine.

Dans la cellule, on introduit 10 cm3 d'une solution contenant :

| | |
|---|---|
| - substrat utilisé dans l'exemple 1 | 51,5 mg |
| - acide acétique | 1M |
| - acétate de sodium (CH₃COONa,3H₂O) | 0,1M |
| - méthanol | q.s.p. 10 cm3 |

Après désaération pendant 10 minutes par barbotage d'un courant d'argon, le potentiel de la cathode est fixé à -1,4 volt par rapport à l'électrode de référence.

On électrolyse la solution pendant le temps nécessaire au passage de 50,4 coulombs.

Après traitement de l'électrolysat dans les conditions décrites à l'exemple 1, on obtient le "Taxotère" avec un rendement de 31 %.

### EXEMPLE 3

On opère dans une cellule d'électrolyse identique à celle décrite dans l'exemple 1.

Dans le compartiment cathodique, on introduit 10 cm3 d'une solution contenant :

| | |
|---|---|
| - substrat utilisé dans l'exemple 1 | 202,1 mg |
| - acide acétique | 1M |
| - acétate de sodium (CH₃COONa,3H₂O) | 0,1M |
| - méthanol | q.s.p. 10 cm3 |

Dans le compartiment anodique, on introduit 10 cm3 du même mélange ne contenant pas le substrat.

Après désaération pendant 10 minutes par barbotage d'un courant d'argon, l'intensité du courant est fixée au départ à 1A/dm2. On électrolyse la solution pendant le temps nécessaire au passage de 181 coulombs.

A l'électrolysat séparé, on ajoute 20 cm3 d'eau permutée. Après évaporation du solvant sous pression réduite à une température inférieure à 35°C, on filtre le précipité formé sur verte fritté N° 3. Après rinçage à l'eau et séchage, on obtient 111,4 mg de solide blanc que l'on purifie par chromatographie sur 50 g de silice (0,063-0,2 mm) contenus dans une colonne de 2 cm de diamètre [éluant : dichlorométhane-méthanol (93-3 en volumes)] en recueillant des fractions d'environ 10 cm3. Les fractions 14 à 40 sont réunies et concentrées à sec sous pression réduite (0,27 kPa) à une température inférieure à 35°C.

On obtient le "Taxotère" avec un rendement de 43 %.

### EXEMPLE 4

Dans le compartiment cathodique d'une cellule d'électrolyse de 25 cm3, en forme de H, divisée en deux compartiments par une membrane échangeuse de cations et contenant :
- une cathode de carbone vitreux dont la surface utile est de 4,5 cm2, et
- une anode de platine,
on introduit 20 cm3 d'une solution contenant :

| | |
|---|---|
| - substrat utilisé dans l'exemple 1 | 205,5 mg |
| - acide acétique | 1M |
| - acétate de tétraéthylammonium | 0,1M |
| - acétonitrile | q.s.p. 20 cm3 |

Dans le compartiment anodique, on introduit 20 cm3 du même mélange ne contenant pas le substrat.

Après désaération pendant 10 minutes par barbotage d'un courant d'argon, l'intensité du courant est fixée à 40 mA puis réduite à 10 mA au-delà du passage de 103 coulombs (0,9 A/dm2).

On électrolyse la solution pendant le temps nécessaire au passage de 150 coulombs.

Le solvant de l'électrolysat est évaporé sous pression réduite à une température inférieure à 35°C. On ajoute 20 cm3 d'eau au résidu puis on extrait par 3 fois 20 cm3 de dichlorométhane. Après séchage de la phase organique sur chlorure de magnésium, on isole 146 mg de produit brut que l'on chromatographie dans les conditions décrites dans l'exemple 3. On obtient le "Taxotère" avec un rendement de 41 %.

## Revendications

1. Procédé de préparation d'un dérivé du taxane de formule générale : 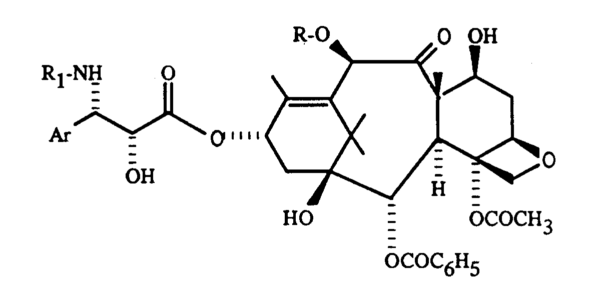 dans laquelle R représente un atome d'hydrogène ou un radical acétyle, R₁ représente un radical benzoyle ou un radical R₂-O-CO- dans lequel R₂ représente un radical alcoyle, alcényle, alcynyle, cycloalcoyle, cycloalcényle, bicycloalcoyle, phényle ou hétérocyclyle azoté et Ar représente un radical aryle caractérisé en ce que l'on effectue la réduction électrolytique d'un produit de formule générale : 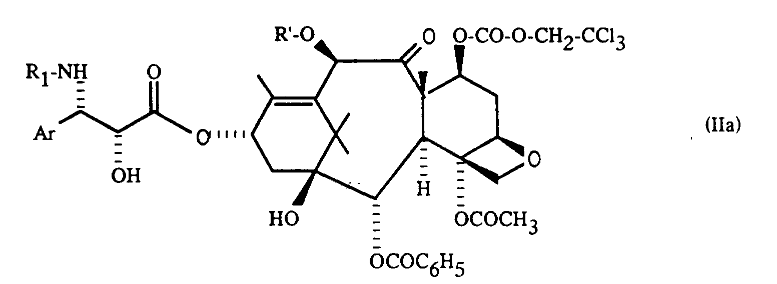 dans laquelle Ar et R₁ sont définis comme précédemment, R' représente un radical acétyle ou un radical trichloro-2,2,2 éthoxycarbonyle.

2. Procédé selon la revendication 1 caractérisé en ce que l'électrolyte est constitué d'un sel alcalin ou d'un sel d'ammonium quaternaire soluble dans le solvant ou dans un mélange hydroorganique.

3. Procédé selon la revendication 2 caractérisé en ce que le solvant est choisi parmi les alcools et les nitriles.

4. Procédé selon la revendication 3 caractérisé en ce que le solvant est choisi parmi le méthanol et l'acétonitrile.

5. Procédé selon l'une des revendications 1 à 4 caractérisé en ce que l'électrolyse est effectuée dans un électrolyseur contenant une cathode choisie parmi le mercure, le zinc, le plomb et le carbone vitreux et une anode choisie parmi le platine, le graphite ou le carbone vitreux.

6. Procédé selon l'une des revendications 1 à 4 caractérisé en ce que l'électrolyse est effectuée dans un électrolyseur, de préférence, à diaphragme.

7. Procédé selon la revendication 6 caractérisé en ce que le diaphragme est constitué par un matériau poreux ou par une membrane échangeuse de cations.

8. Procédé selon l'une des revendications 1 à 7 caractérisé en ce que l'électrolyse est effectuée à potentiel contrôlé.

9. Procédé selon l'une des revendications 1 à 7 caractérisé en ce que l'électrolyse est effectuée à intensité de courant constante.

10. Procédé selon la revendication 1 pour la préparation du tert.butoxycarbonylamino-3 phényl-3 hydroxy-2 propanoate-(2R,3S) d'acétoxy-4 benzoyloxy-2α époxy-5β,20 trihydroxy-1,7β,10β oxo-9 taxène-11 yle-13α ou "Taxotère".

## Claims

1. Process for the preparation of a taxane derivative of general formula: 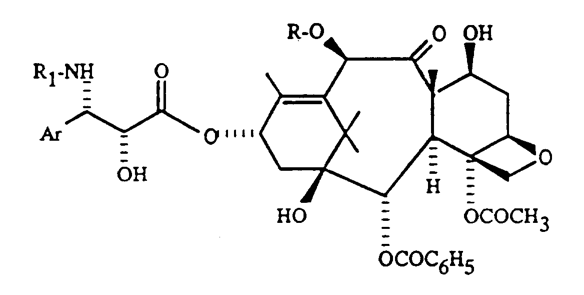 in which R represents a hydrogen atom or an acetyl radical, R₁ represents a benzoyl radical or a radical R₂-O-CO- in which R₂ represents an alkyl, alkenyl, alkynyl, cycloalkyl, cycloalkenyl, bicycloalkyl, phenyl or nitrogen containing heterocyclyl radical and Ar represents an aryl radical, characterised in that a product of general formula: 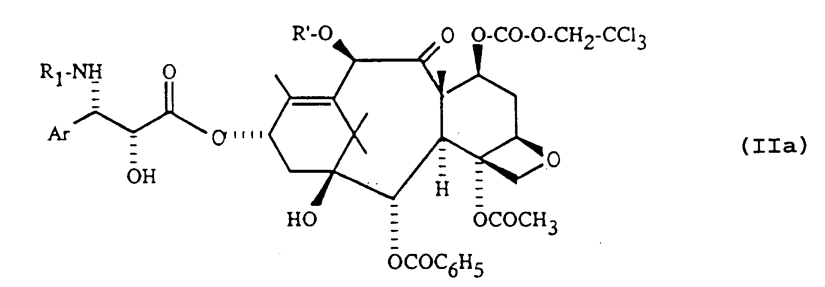 in which Ar and R₁ are defined as above and R' represents an acetyl radical or a 2,2,2-trichloroethoxycarbonyl radical, is electrolytically reduced.

2. Process according to claim 1, characterised in that the electrolyte consists of an alkali metal salt or a quaternary ammonium salt which is soluble in the solvent or in an aqueous/organic mixture.

3. Process according to claim 2, characterised in that the solvent is chosen from the alcohols and the nitriles.

4. Process according to claim 3, characterised in that the solvent is chosen from methanol and acetonitrile.

5. Process according to one of claims 1 to 4, characterised in that the electrolysis is carried out in an electrolyser containing a cathode chosen from mercury, zinc, lead and vitreous carbon and an anode chosen from platinum, graphite or vitreous carbon.

6. Process according to one of claims 1 to 4, characterised in that the electrolysis is carried out in an electrolyser, preferably a diaphragm electrolyser.

7. Process according to claim 6, characterised in that the diaphragm consists of a porous material or of a cation-exchange membrane.

8. Process according to one of claims 1 to 7, characterised in that the electrolysis is carried out with controlled potential.

9. Process according to one of claims 1 to 7, characterised in that the electrolysis is carried out with constant current strength.

10. Process according to claim 1 for the preparation of 4-acetoxy-2α-benzoyloxy-5β,20-epoxy-1,7β,10β-trihydroxy-9-oxo-11-taxen-13α-yl (2R,3S)-3-(tert-butoxycarbonylamino)-3-phenyl-2-hydroxypropanoate or "Taxotere".

## Patentansprüche

1. Verfahren zur Herstellung eines Taxanderivates der allgemeinen Formel 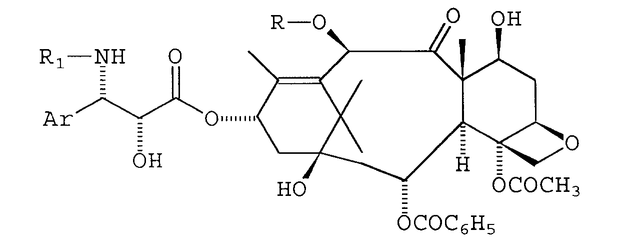 in der R ein Wasserstoffatom oder einen Acetylrest darstellt, R₁ einen Benzoylrest oder einen Rest R₂-O-CO- bedeutet, worin R₂ einen der Reste Alkyl, Alkenyl, Alkinyl, Cycloalkyl, Cycloalkenyl, Bicycloalkyl, Phenyl oder Stickstoff-Heterocyclyl darstellt, und Ar ein Arylrest ist, dadurch gekennzeichnet, daß man die elektrolytische Reduktion eines Produktes der allgemeinen Formel (IIa) 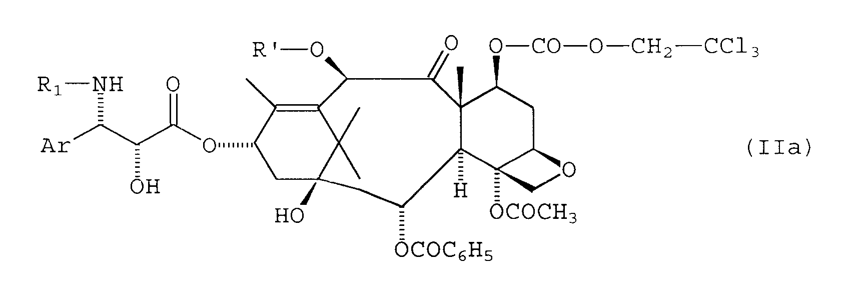 durchführt, in der Ar und R₁ wie vorstehend definiert sind und R' einen Acetylrest oder einen 2,2,2-Trichlor-ethoxycarbonylrest darstellt.

2. Verfahren nach Anspruch 1, dadurch gekennzeichnet, daß der Elektrolyt aus einem Alkalisalz oder aus einem quaternären Ammoniumsalz gebildet wird, das in dem Lösungsmittel oder in einer hydroorganischen Mischung löslich ist.

3. Verfahren nach Anspruch 2, dadurch gekennzeichnet, daß das Lösungsmittel unter den Alkoholen und den Nitrilen ausgewählt wird.

4. Verfahren nach Anspruch 3, dadurch gekennzeichnet, daß das Lösungsmittel unter Methanol und Acetonitril ausgewählt wird.

5. Verfahren nach einem der Ansprüche 1 bis 4, dadurch gekennzeichnet, daß die Elektrolyse in einem Elektrolysator durchgeführt wird, der eine Kathode, ausgewählt unter Quecksilber, Zink, Blei und Glaskohlenstoff, und eine Anode, ausgewählt unter Platin, Graphit oder Glaskohlenstoff, enthält.

6. Verfahren nach einem der Ansprüche 1 bis 4, dadurch gekennzeichnet, daß die Elektrolyse in einem Elektrolysator, vorzugsweise mit Diaphragma durchgeführt wird.

7. Verfahren nach Anspruch 6, dadurch gekennzeichnet, daß das Diaphragma aus einem porösen Material oder aus einer Kationenaustauscher-Membran besteht.

8. Verfahren nach einem der Ansprüche 1 bis 7, dadurch gekennzeichnet, daß die Elektrolyse unter geregelter Spannung durchgeführt wird.

9. Verfahren nach einem der Ansprüche 1 bis 7, dadurch gekennzeichnet, daß die Elektrolyse bei einer konstanten Stromstärke durchgeführt wird.

10. Verfahren nach Anspruch 1 zur Herstellung von 3-tert.-Butoxycarbonylamino-3-phenyl-2-hydroxy-(2R,3S)-propanoat von 4-Acetoxy-2α-benzoyloxy-5β,20-epoxy-1,7β10β-trihydroxy-9-oxo-11-taxen-13α-yl oder "Taxoter".
